Europäisches Patentamt

European Patent Office

Office européen des brevets

(19)

(11) Publication number: **0 513 162 B1**

(12) **EUROPEAN PATENT SPECIFICATION**

(45) Date of publication of patent specification: **22.11.95**  (51) Int. Cl.⁶: **C07C 5/393**

(21) Application number: **91903947.9**

(22) Date of filing: **09.04.90**

(86) International application number:
**PCT/US90/02027**

(87) International publication number:
**WO 91/11416 (08.08.91 91/18)**

(54) PROCESS FOR DEHYDROGENATION/DEHYDROCYCLIZATION OF ALIPHATICS.

(30) Priority: **24.01.90 US 469648**
**24.01.90 US 469650**
**24.01.90 US 469651**

(43) Date of publication of application:
**19.11.92 Bulletin 92/47**

(45) Publication of the grant of the patent:
**22.11.95 Bulletin 95/47**

(84) Designated Contracting States:
**AT BE CH DE DK ES FR GB IT LI LU NL SE**

(56) References cited:
**US-A- 4 329 532**
**US-A- 4 804 801**
**US-A- 4 826 667**
**US-A- 4 888 105**

**No further relevant documents disclosed**

(73) Proprietor: **MOBIL OIL CORPORATION**
**150 East 42nd Street**
**New York**
**New York 10017 (US)**

(72) Inventor: **CHU, Cynthia, Ting-Wah**
**18 Indian Run Road**
**Princeton Junction, NJ 08550 (US)**
Inventor: **DEGNAN, Thomas Francis, Jr.**
**40 North Homestead Drive**
**Yardley, PA 19067 (US)**
Inventor: **DEL ROSSI, Kenneth, Joseph**
**608 Foxton Court**
**Mantua, NJ 08051 (US)**
Inventor: **DESSAU, Ralph, Moritz**
**14 Cellar Road**
**Edison, NJ 08817 (US)**
Inventor: **HUH, Billy, Keon**
**5005 Little Turtle Drive,**
**Birmingham, Alabama 35242 (US)**
Inventor: **HUSS, Albin, Jr.**
**51 Stirling Way**
**Chadds Ford, PA 19317 (US)**
Inventor: **KIRKER, Garry, Wayne**
**18 Old Mill Road**
**Sewell, NJ 08080 (US)**
Inventor: **MARLER, David, Owen**
**801 Cooper Street, 272 B**
**Deptford, NJ 08096 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 513 162 B1

Inventor: **PARTRIDGE, Randall, David**
**58 Jacobs Creek Road**
**West Trenton, NJ 08628 (US)**

(74) Representative: **Colmer, Stephen Gary et al**
**European Office of Patent Counsel**
**Mobil House**
**500-600 Witan Gate**
**Central Milton Keynes**
**Buckinghamshire MK9 1ES (GB)**

## Description

This invention relates to a process for dehydrogenation and/or dehydrocyclization of a feedstock containing $C_2$ to $C_{12}$ aliphatic hydrocarbons, e.g., a Udex raffinate, particularly to produce aromatics or mixture of aromatics and olefins, in the presence of a zeolite catalyst.

Zeolitic materials, both natural and synthetic, have been demonstrated in the past to have catalytic properties for various types of hydrocarbon conversion. Certain zeolitic materials are ordered, porous crystalline aluminosilicates having a definite crystalline structure as determined by X-ray diffraction, within which there are a large number of smaller cavities which may be interconnected by a number of still smaller channels or pores. These cavities and pores are uniform in size within a specific zeolitic material. Since the dimensions of these pores are such as to accept for adsorption molecules of certain dimensions while rejecting those of larger dimensions, these materials have come to be known as "molecular sieves" and are utilized in a variety of ways to take advantage of these properties. Such molecular sieves, both natural and synthetic, include a wide variety of positive ion-containing crystalline silicates. These silicates can be described as a rigid three-dimensional framework of $SiO_4$ and Periodic Table Group IIIA element oxide, e.g., $AlO_4$, in which the tetrahedra are cross-linked by the sharing of oxygen atoms whereby the ratio of the total Group IIIA element, e.g., aluminum, and silicon atoms to oxygen atoms is 1:2. The electrovalence of the tetrahedra containing the Group IIIA element, e.g., aluminum, is balanced by the inclusion in the crystal of a cation, e.g., an alkali metal or an alkaline earth metal cation. This can be expressed wherein the ratio of the Group IIA element, e.g., aluminum, to the number of various cations, such as Ca/2, Sr/2, Na, K or Li, is equal to unity. One type of cation may be exchanged either entirely or partially with another type of cation utilizing ion exchange techniques in a conventional manner. By means of such cation exchange, it has been possible to vary the properties of a given silicate by suitable selection of the cation.

Prior art techniques have resulted in the formation of a great variety of synthetic zeolites. Many of these zeolites have come to be designated by letter or other convenient symbols, as illustrated by zeolite Z (U.S. Patent No. 2,882,243), zeolite X (U.S. Patent No. 2,882,244), zeolite Y (U.S. Patent No. 3,130,007), zeolite ZK-5 (U.S. Patent No. 3,247,195), zeolite ZK-4 (U.S. Patent No. 3,314,752), zeolite ZSM-5 (U.S. Patent No. 3,702,886), zeolite ZSM-11 (U.S. Patent No. 3,709,979), zeolite ZSM-12 (U.S. Patent No. 3,832,449), zeolite ZSM-20 (U.S. Patent No. 3,972,983), zeolite ZSM-35 (U.S. Patent No. 4,016,245), and zeolite ZSM-23 (U.S. Patent No. 4,076,842).

The $SiO_2/Al_2O_3$ ratio of a given zeolite is often variable. For example, zeolite X can be synthesized with $SiO_2/Al_2O_3$ ratios of from 2 to 3; zeolite Y, from 3 to 6. In some zeolites, the upper limit of the $SiO_2/Al_2O_3$ ratio is unbounded. ZSM-5 is one such example wherein the $SiO_2/Al_2O_3$ ratio is at least 5 and up to the limits of present analytical measurement techniques. U.S. Patent No. 3,941,871 (Re. 29,948) discloses a porous crystalline silicate made from a reaction mixture containing no deliberately added alumina in the recipe and exhibiting the X-ray diffraction pattern characteristic of ZSM-5. U.S. Patent Nos. 4,061,724, 4,073,865 and 4,104,294 describe crystalline silicates of varying alumina and metal content.

Zeolites have been used in the past in the production of aromatic hydrocarbons from aliphatic hydrocarbons. For example, U.S. Patent No. 3,756,942 discloses a process for converting paraffinic feedstocks over zeolites such as ZSM-5 to produce a variety of hydrocarbon products. The underlying chemistry involved in this conversion is extremely complex. More particularly, a number of simultaneous and sometimes competing reactions take place to produce a variety of products which can, in turn, be reacted to form still different products. These possible reactions include cracking of paraffins, aromatization of olefins, and alkylation and dealkylation of aromatics. Products from the conversion of $C_5^+$ paraffinic feedstocks over ZSM-5 include $C_6$-$C_8$ aromatics, $C_2$-$C_4$ olefins, $C_9$ + aromatics and $C_1$-$C_3$ paraffins. Of these products the $C_6$-$C_8$ aromatics and $C_2$-$C_4$ olefins are the most desired. $C_6$-$C_8$ aromatics, e.g., benzene, toluene, xylene and ethylbenzene, also referred to collectively as BTX, are valuable organic chemicals which can be used in a variety of ways. Since BTX has a high octane value it can be used as a blending stock for making high octane gasoline. $C_2$-$C_4$ olefins, e.g., ethylene, propylene and butene, are also valuable organic chemicals which can be used to form polymers. By way of contrast, $C_1$-$C_3$ paraffins (i.e., methane, ethane and propane), particularly in admixture, are less valuable chemicals which are generally used for fuel.

According to U.S. Patent No. 3,755,486, $C_{6-10}$ hydrocarbons undergo dehydrocyclization to benzene and alkylbenzenes in the presence of a Li, Na or K zeolite X or Y or faujasite impregnated with 0.3 to 1.4 percent Pt.

U.S. Patent No. 3,760,024 discloses the aromatization of a feed containing $C_{2-4}$ paraffins and/or olefins in the absence of added hydrogen employing ZSM-5 as catalyst.

According to U.S. Patent No. 3,855,115, aromatization of hydrocarbons is accomplished employing rhenium-exchanged ZSM-5.

Aliphatic naphthas are upgraded to products of increased aromatics content by the process disclosed in U.S. Patent No. 3,890,218. The process employs a zeolite catalyst such as ZSM-5 into which one or more metals which increase the aromatization activity of the zeolite, e.g., zinc or cadmium, have been incorporated.

In the process disclosed in U.S. Patent No. 4,347,394, light straight-run naphthas and similar mixtures are converted to highly aromatic mixtures, principally benzene, employing a Group VIII metal-containing intermediate pore size zeolite, e.g., ZSM-5, which has been rendered substantially free of acidity by treatment with an alkali metal compound, e.g., NaOH.

Gaseous feedstocks containing ethane are converted to a mixture of benzene, toluene and xylene ("BTX") in the process of U.S. Patent No. 4,350,835 utilizing a gallium-containing zeolite such as ZSM-5. A similar catalyst further containing thorium is disclosed in U.S. Patent No. 4,629,818.

U.S. Patent No. 4,435,283 describes a method for dehydrocyclizing alkanes employing as catalyst, a Group VIII metal-containing large pore zeolite which further contains an alkaline earth metal, e.g., zeolite X, Y or L containing Pt and barium.

In accordance with the process disclosed in U.S. Patent No. 4,720,602, $C_2$ to $C_{12}$ aliphatic hydrocarbons are converted to aromatics over a zeolite catalyst, e.g., ZSM-5, which has been activated with zinc.

According to the present invention, there is provided a process for dehydrogenation and/or dehydrocyclization of a feedstock containing at least one $C_2$-$C_{12}$ aliphatic hydrocarbon which comprises contacting the feedstock with a conversion catalyst comprising a synthetic porous crystalline zeolite which, in its calcined form, has an X-ray diffraction pattern including the values listed in Table I below:

TABLE I

| Interplanar d-Spacing (A) | Relative Intensity, I/Io x 100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS |

More specifically, the calcined form may be characterized by an X-ray diffraction pattern including the following lines:

TABLE II

| Interplanar d-Spacing (A) | Relative Intensity, I/Io x 100 |
|---|---|
| $30.0 \pm 2.2$ | W-M |
| $22.1 \pm 1.3$ | W |
| $12.36 \pm 0.4$ | M-VS |
| $11.03 \pm 0.2$ | M-S |
| $8.83 \pm 0.14$ | M-VS |
| $6.86 \pm 0.14$ | W-M |
| $6.18 \pm 0.12$ | M-VS |
| $6.00 \pm 0.10$ | W-M |
| $5.54 \pm 0.10$ | W-M |
| $4.92 \pm 0.09$ | W |
| $4.64 \pm 0.08$ | W |
| $4.41 \pm 0.08$ | W-M |
| $4.25 \pm 0.08$ | W |
| $4.10 \pm 0.07$ | W-S |
| $4.06 \pm 0.07$ | W-S |
| $3.91 \pm 0.07$ | M-VS |
| $3.75 \pm 0.06$ | W-M |
| $3.56 \pm 0.06$ | W-M |
| $3.42 \pm 0.06$ | VS |
| $3.30 \pm 0.05$ | W-M |
| $3.20 \pm 0.05$ | W-M |
| $3.14 \pm 0.05$ | W-M |
| $3.07 \pm 0.05$ | W |
| $2.99 \pm 0.05$ | W |
| $2.82 \pm 0.05$ | W |
| $2.78 \pm 0.05$ | W |
| $2.68 \pm 0.05$ | W |
| $2.59 \pm 0.05$ | W |

These values were determined by standard techniques. The radiation was the K-alpha doublet of copper and a diffractometer equipped with a scintillation counter and an associated computer was used. The peak heights, I, and the positions as a function of 2 theta, where theta is the Bragg angle, were determined using algorithms on the computer associated with the diffractometer. From these, the relative intensities, 100 $I/I_o$, where $I_o$ is the intensity of the strongest line or peak, and d (obs.) the interplanar spacing in Angstroms Units (A), corresponding to the recorded lines, were determined. In Tables I and II, the relative intensities are given in terms of the symbols W = weak, M = medium, S = strong and VS = very strong. In terms of intensities, these may be generally designated as follows:

$$W = 0 - 20$$
$$M = 20 - 40$$
$$S = 40 - 60$$
$$VS = 60 - 100$$

It should be understood that these X-ray diffraction patterns are characteristic of all species of the zeolite. The sodium form as well as other cationic forms reveal substantially the same pattern with some minor shifts in interplanar spacing and variation in relative intensity. Other minor variations can occur depending on the Y to X, e.g., silicon to aluminum, mole ratio of the particular sample, as well as its degree of thermal treatment.

The zeolite employed as the catalyst in the process of the invention, generally has a composition involving the molar relationship:

$$X_2O_3:(n)YO_2,$$

wherein X is a trivalent element, such as aluminum, boron, iron and/or gallium, preferably aluminum, Y is a tetravalent element such as silicon and/or germanium, preferably silicon, and n is at least 10, usually from 10 to 150, more usually from 10 to 60, and even more usually from 20 to 40. In the as-synthesized form, the zeolite has a formula, on an anhydrous basis and in terms of moles of oxides per n moles of $YO_2$, as follows:

$$(0.005-0.1)Na_2O:(1-4)R:X_2O_3:nYO_2$$

wherein R is an organic. The Na and R components are associated with the zeolite as a result of their presence during crystallization, and are easily removed by conventional post-crystallization methods.

The zeolite employed herein is thermally stable and exhibits high surface area (greater than 400 $m^2/gm$ as measured by the BET [Bruenauer, Emmet and Teller] test) and unusually large sorption capacity when compared to similar crystal structures. In particular, the zeolite exhibits Equilibrium Adsorption values greater than 4.5 wt.%, usually greater than 7 wt.% for cyclohexane vapor, greater than 10 wt.% for n-hexane vapor, and normally greater than 10 wt.% for water vapor. As is evident from the above formula, the present is synthesized nearly free of Na cations. It can, therefore, be used as a catalyst with acid activity without an exchange step. To the extent desired, however, the original sodium cations of the as-synthesized material can be replaced in accordance with techniques well known in the art, at least in part, by ion exchange with other cations. Preferred replacing cations include metal ions, hydrogen ions, hydrogen precursor, e.g., ammonium, ions and mixtures thereof. Particularly preferred cations are those which tailor the activity of the catalyst for the conversion process herein. These include hydrogen, rare earth metals and metals of Groups IIA, IIIA, IVA, IB, IIB, IIIB, IVB and VIII of the Periodic Table of the Elements.

Prior to its use as catalyst herein, the zeolite should be subjected to thermal treatment to remove part or all of any organic constituent present therein.

The zeolite catalyst used herein can also be used in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be introduced in the catalyst composition by way of cocrystallization, exchanged into the composition to the extent a Group IIIA element, e.g., aluminum, is in the structure, impregnated therein or intimately physically admixed therewith. Such component can be impregnated in, or on, the zeolite such as, for example, by, in the case of platinum, treating the zeolite with a solution containing a platinum metal-containing ion. Thus, suitable platinum compounds for this purpose include chloroplatinic acid, platinous chloride ad various compounds containing the platinum amine complex.

Prior to its use in the process of this invention, the zeolite should be dehydrated, at least partially. This can be done by heating the zeolite to a temperature in the range of from 200°C to 595°C in an atmosphere such as air and nitrogen at atmospheric, subatmospheric or superatmospheric pressures for between 30 minutes to 48 hours. Dehydration can also be performed at room temperature merely by placing the crystalline material in a vacuum, but a longer time is required to obtain a sufficient amount of dehydration.

The zeolite employed in the present invention can be prepared from a reaction mixture containing sources of alkali or alkaline earth metal (M), e.g., sodium or potassium, cation, an oxide of trivalent element X, e.g, aluminum, an oxide of tetravalent element Y, e.g., silicon, an organic (R) directing agent in the form of hexamethyleneimine and water, said reaction mixture having a composition, in terms of mole ratios of oxides, within the following ranges:

| Reactants | Useful | Preferred |
|---|---|---|
| $YO_2/X_2O_3$ | 10 - 60 | 10 - 40 |
| $H_2O/YO_2$ | 5 - 100 | 10 - 50 |
| $OH^-/YO_2$ | 0.01 - 1.0 | 0.1 - 0.5 |
| $M/YO_2$ | 0.01 - 2.0 | 0.1 - 1.0 |
| $R/YO_2$ | 0.05 - 1.0 | 0.1 - 0.5 |

In a preferred synthesis method, the $YO_2$ reactant contains a substantial amount of solid $YO_2$, e.g., at least 30 wt.% solid $YO_2$. Where $YO_2$ is silica, the use of a silica source containing at least 30 wt.% solid silica, e.g., Ultrasil (a precipitated, spray dried silica containing 90 wt.% silica) or HiSil (a precipitated hydrated $SiO_2$ containing 87 wt.% silica, 6 wt.% free $H_2O$ and 4.5 wt.% bound $H_2O$ of hydration and having a particle size of 0.02 micron) favors crystal formation from the above mixture. If another source of oxide of silicon, e.g., Q-Brand (a sodium silicate comprised of 28.8 wt.% of $SiO_2$, 8.9 wt.% $Na_2O$ and 62.3 wt.%

$H_2O$) is used, crystallization may yield little if any of the required zeolite and impurity phases of other crystal structures, e.g., ZSM-12, may be produced. Preferably, therefore, the $YO_2$, e.g., silica, source contains at least 30 wt.% solid $YO_2$, e.g., silica, and more preferably at least 40 wt.% solid $YO_2$, e.g., silica.

Crystallization can be carried out at either static or stirred conditions in a suitable reactor vessel, such as, e.g., polypropylene jars or teflon lined or stainless steel autoclaves. Crystallization is generally conducted at 80°C to 225°C for 25 hours to 60 days. Thereafter, the crystals are separated from the liquid and recovered.

Crystallization is facilitated by the presence of at least 0.01 percent, preferably 0.10 percent and still more preferably 1 percent, seed crystals based on total weight of the crystalline product.

Prior to use in the process of the invention, the zeolite is preferably combined with another material which is resistant to the temperatures and other conditions employed in the process of this invention. Such materials include active and inactive materials and synthetic or naturally occurring zeolites as well as inorganic materials such as clays, silica and/or metal oxides such as alumina. The latter may be either naturally occurring or in the form of gelatinous precipitates or gels including mixtures of silica and metal oxides. Use of a material in conjunction with the instant zeolite, i.e., combined therewith or present during its synthesis, which itself is catalytically active may change the conversion and/or selectivity of the catalyst. Inactive materials suitably serve as diluents to control the amount of conversion so that products can be obtained economically and orderly without employing other means for controlling the rate of reaction. These materials may be incorporated into naturally occurring clays, e.g., bentonite and kaolin, to improve the crush strength of the catalyst under commercial operating conditions. Said materials, i.e., clays, oxides, etc., function as binders for the catalyst. It is desirable to provide a catalyst having good crush strength because in commercial use, it is desirable to prevent the catalyst from breaking down into powder-like materials. These clay binders have been employed normally only for the purpose of improving the crush strength of the catalyst.

Naturally occurring clays which can be composited with ther present crystals include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with the zeolite also include inorganic oxides, notably alumina.

In addition to the foregoing materials, the present zeolite can be composited with a porous matrix material such as silica-alumina, silica-magnesia, silica-zirconia, silica-thoria, silica-beryllia, silica-titania as well as ternary compositions such as silica-alumina-thoria, silica-alumina-zirconia silica-alumina-magnesia and silica-magnesia-zirconia. It may also be advantageous to provide at leat a part of the foregoing matrix materials in colloidal form so as to facilitate extrusion of the bound catalyst component(s).

The relative proportions of zeolite and inorganic oxide matrix vary widely, with the crystal content ranging from 1 to 90 percent by weight and more usually, particularly when the composite is prepared in the form of beads, in the range of 2 to 80 weight percent of the composite.

The stability of the catalyst of the invention may be increased by steaming, which is conveniently effected by contacting the zeolite with 5-100% steam at a temperature of at least 300°C (preferably 300-650°C) for at least one hour (preferably 1-200 hours) at a pressure of 101-2,500 kPa. In a more particular embodiment, the catalyst can be made to undergo steaming with 75-100% steam at 315°-500°C and atmospheric pressure for 2-25 hours.

The feed stream to the process of this invention contains at least 20% and more preferably at least 50% by weight of at least one aliphatic hydrocarbon containing 2 to 12 carbon atoms. The hydrocarbon may be straight chain, open chain or cyclic and may be saturated or unsaturated. Some contemplated hydrocarbons are ethane, propane, propylene, n-butane, n-butenes, isobutane, isobutene, straight chain, branched chain and cyclic pentanes, pentenes, hexanes, hexenes, heptanes, heptenes, octanes, octenes, nonanes, nonenes, decanes, undecanes, decenes, undecenes, dodecanes and dodecenes. A particularly useful hydrocarbon feedstock herein is a raffinate from a hydrocarbon mixture which has had aromatics removed therefrom by a solvent extraction treatment. Examples of such solvent extraction treatments are described on pages 706-709 of the Kirk-Othmer Encyclopedia of Chemical Technology, Third Edition, Vol. 9, John Wiley and Sons, 1980. One such hydrocarbon feedstock is a Udex raffinate, a typical composition of which is as follows:

| Component | Wt. Percent |
|---|---|
| $C_5$ | 6.20 |
| $C_5^=$ | 0.19 |
| $C_6$ | 45.80 |
| $C_6^=$ | 8.49 |
| $C_7$ | 27.93 |
| $C_7^=$ | 3.56 |
| $C_8$s | 1.87 |
| Benzene | 0.39 |
| Toluene | 3.85 |
| EB | 0.34 |
| Xylene | 0.39 |
| $C_9^+$ Aromatics | 1.18 |

Another suitable feedstream is a straight run, thermal or hydrocracked naphtha or any other naphtha containing one or more $C_2$-$C_{12}$ paraffins. Naphthas exhibit boiling point temperature ranges of up to 400°F (204°C) with light naphtha fractions exhibiting a boiling point temperature range of from 80 to 250°F (27 to 120°C).

The hydrocarbon conversion process of this invention is conducted by contacting the feedstock with the above zeolite in a reaction zone, such as, for example, a fixed or fluid bed of catalyst composition under effective conversion conditions. In a typical embodiment of the process of this invention, the feed stream is introduced into the reaction zone at a temperature within the range of 316°C (600°F) to 760°C (1400°F), a pressure within the range of atmospheric to 2860 kPa (400 psig) and a liquid hourly space velocity (LHSV) of 0.1 to 100. More preferably, the temperature employed is 400 to 680°C (750-1250°F), most preferably 450-590°C (800-1100°F), the pressure is 445-1825 kPa (50-250 psig) and the LHSV is 0.5-5.

The aromatics selectivity of the catalyst of the invention appears to be influenced by the acid activity of the zeolite. One measure of acid activity is alpha value, which is an approximate indication of the catalytic cracking activity of the zeolite compared to that of a standard catalyst. Alpha Value expresses the relative rate constant (rate of normal hexane conversion per volume of catalyst per unit time) compared to silica-alumina cracking catalyst taken as having an Alpha Value of 1 (rate Constant = 0.016 sec$^{-1}$). The test for measuring Alpha Value used herein is described in J. Catalysis, 61, pp. 390-396 (1980).

Thus, in a first embodiment of the present invention, the zeolite employed has an alpha value not exceeding 150. Preferably, the Alpha Value of the zeolite does not exceed 50 and more preferably, does not exceed 10. However, the zeolite of the first embodiment should possess some acidity, such that when 1gm of the finished zeolite catalyst is slurried in 100 ml of water, it exhibits a pH of less than 8 and preferably less than 7.

Producing a zeolite with such a low Alpha Value can be effected by a variety of techniques including (a) synthesizing the zeolite with a high silica/aluina ratio, (b) steaming, (c) steaming followed by dealumination and (d) substituting framework aluminum. with other trivalent metal species, for example, boron. In the case of steaming, the zeolite can be exposed to steam at elevated temperatures ranging from 260 to 650°C (500 to 1200°F) and preferably 400 to 540°C (750 to 1000°F). This treatment can be accomplished in an atmosphere of 100% steam or an atmosphere consisting of steam and a gas which is substantially inert to the zeolite. A similar treatment can be accomplished at lower temperatures employing elevated pressure, e.g., at 180 to 370°C (350 to 700°F) and from 1000 to 20200 kPa (10 to 200 atmospheres). Specific details of several steaming procedures can be gained from the disclosures of U.S. Patent No.s 4,325,994; 4,374,296; and 4,418,235.

Aside from, or in addition to any of the foregoing procedures, the acidity of the zeolite can be reduced by treatment with a base material, e.g., a hydroxide or basic salt of an alkali metal or alkaline earth metal such as dosium hydroxide, sodium carbonate, sodium bicarbonate, sodium nitrate, potassium hydroxide, potassium carbonate, calcium hydroxide, calcium carbonate, barium hydroxide and barium carbonate.

In a second embodiment the zeolite employed is such that it exhibits essentially no acid activity. The expression "essentially non-acidic" refers to a finished form of the catalyst, i.e., the zeolite including the Group VIII metal species component (referred to hereinafter) as well as any optional binder component, exhibiting a pH of at least 8, and preferably of at least 9, as measured when 1gm of the catalyst is slurried in 100 ml of water. Stated in functional or catalytic terms, the expression "essentially non-acidic" refers to a form of the zeolite which when used to catalyze the dehydrocyclization conversion of n-octane to xylenes, produces an amount of o-xylene which exceeds the equilibrium amount of o-xylene under the conversion conditions selected.

An essentially non-acidic form of the zeolite employed herein can be obtained by reducing the acid content of the zeolite by ion exchange with Group IA and/or IIA cations, preferably subsequent to inclusion of the Group VIII metal species. Suitable sources of Group IA and Group IIA cations include the hydroxide or basic salts of alkali metals or alkaline earth metals such as sodium hydroxide, sodium carbonate, sodium bicarbonate, potassium hydroxide, potassium carbonate, calcium hydroxide, calcium carbonate, barium hydroxide, barium carbonate and cerium hydroxide.

In the first and second embodiment described above, which employs a low-acidity and an essentially non-acidic zeolite respectively, it is necessary that the final catalyst contains at least one Group VIII metal species. The expression "Group VIII metal species" as used herein contemplates the metal per se, e.g., platinum, palladium, iridium, rhenium and rhodium; combinations of such metals and compounds thereof. The preferred metals are platinum and palladium and of these, platinum is the most preferred. The Group VIII metal component can be physically and/or chemically associated with the zeolite and/or any binder or matrix material with which the zeolite may be composited. For example, the Group VIII metal species can be impregnated into the zeolite after they are formed or the metal can be included in the reaction mixture from which the zeolite is formed. For example, in the case of platinum, the zeolite can be treated with a solution containing a platinum metal-containing ion. Thus, suitable platinum compounds for this purpose include chloroplatinic acid, platinous chloride and any of various compounds containing a platinum amine complex. The amount of Group VIII metal present on the zeolite can vary from 0.01 to 5.0 weight percent, preferably 0.1 to 2.0 weight percent, and most preferably 0.2 to 1.0 weight percent by weight of the zeolite.

In order to further enhance selectivity, the low and no acidity zeolites of the first and second embodiments respectively can be further associated with one or more species of elements such as tin, indium, thallium, lead and/or sulfur.

The invention will now be more particularly described with reference to the following Examples and the accompanying drawings, in which:

Figures 1-5 are X-ray diffraction patterns of the calcined crystalline material products of Examples 1, 3, 4, 5 and 7, respectively: and

Figures 6-8 are graphical representations of process performance data relating to the aliphatic hydrocarbon conversion process of this invention.

In the Examples, unless otherwise indicated, all parts are by weight. Whenever sorption data are set forth for comparison of sorptive capacities for water, cyclohexane and/or n-hexane, they were Equilibrium Adsorption values determined as follows:

A weighed sample of the calcined adsorbent was contacted with the desired pure adsorbate vapor in an adsorption chamber, evacuated to less than 1 mm Hg and contacted with 1.6 kPa (12 Torr) of water vapor or 5.3 kPa (40 Torr) of n-hexane or 5.3 kPa (40 torr) of cyclohexane vapor, pressures less than the vapor-liquid equilibrium pressure of the respective adsorbate at 90°C. The pressure was kept constant (within about ± 0.5 mm Hg) by addition of adsorbate vapor controlled by a manostat during the adsorption period, which did not exceed about 8 hours. As adsorbate was adsorbed by the zeolite crystalline material, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant. The zeolite employed in the process of the invention always exhibits Equilibrium Adsorption values of greater than 10 wt.% for water vapor, greater than 4.5 wt.%, usually greater than 7 wt.% for cyclohexane vapor and greater than 10 wt.% for n-hexane vapor.

When Alpha Value is examined, it is noted that the Alpha Value is an approximate indication of the catalytic cracking activity of the catalyst compared to a standard catalyst and it gives the relative rate constant (rate of normal hexane conversion per volume of catalyst per unit time). It is based on the activity of a highly active silica-alumina cracking catalyst taken as an Alpha of 1 (Rate Constant = 0.016 sec$^{-1}$). The Alpha Test used herein is described in J. Catalysis, 61, pp. 390-396 (1980).

EXAMPLE 1

1 part of sodium aluminate (43.5% $Al_2O_3$, 32.2% $Na_2O$, 25.6% $H_2O$) was dissolved in a solution containing 1 part of 50% NaOH solution and 103.13 parts $H_2O$. To this was added 4.50 parts hexamethyleneimine. The resulting solution was added to 8.55 parts of Ultrasil, a precipitated, spray-dried silica (about 90% $SiO_2$).

The reaction mixture had the following composition, in mole ratios:

| | |
|---|---|
| $SiO_2/Al_2O_3$ | = 30.0 |
| $OH^-/SiO_2$ | = 0.18 |
| $H_2O/SiO_2$ | = 44.9 |
| $Na/SiO_2$ | = 0.18 |
| $R/SiO_2$ | = 0.35 |

where R is hexamethyleneimine.

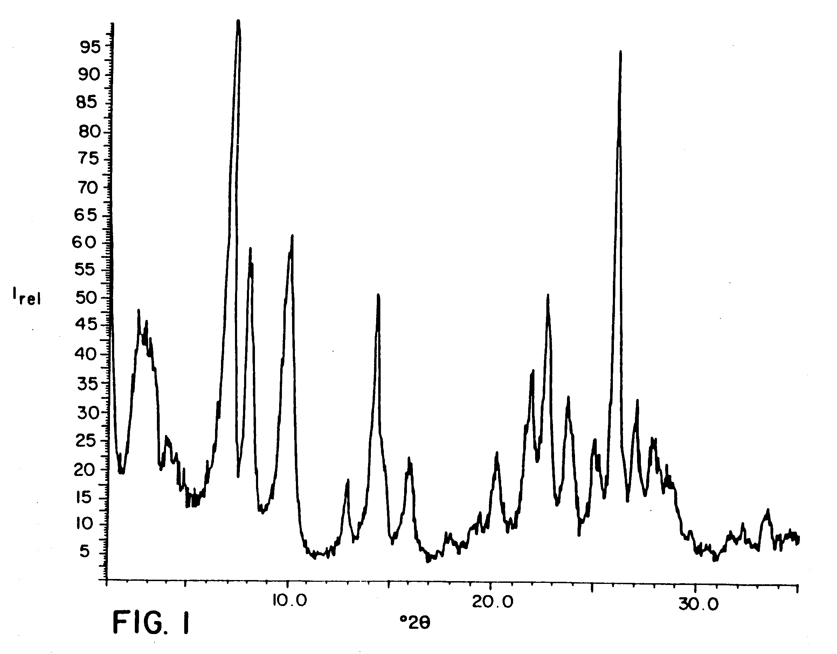

The mixture was crystallized in a stainless steel reactor, with stirring, at 150°C for 7 days. The crystalline product was filtered, washed with water and dried at 120°C. After a 20 hour calcination at 538°C, the X-ray diffraction pattern contained the major lines listed in Table III. Figure 1 shows the X-ray diffraction pattern of the calcined product. The sorption capacities of the calcined material were measured to be:

| | |
|---|---|
| $H_2O$ | 15.2 wt.% |
| Cyclohexane | 14.6 wt.% |
| n-Hexane | 16.7 wt.% |

The surface area of the calcined crystalline material was measured to be 494 $m^2$/g.

The chemical composition of the uncalcined material was determined to be as follows:

| Component | wt.% |
|---|---|
| $SiO_2$ | 66.9 |
| $Al_2O_3$ | 5.40 |
| Na | 0.03 |
| N | 2.27 |
| Ash | 76.3 |
| $SiO_2/Al_2O_3$, mole ratio - 21.1 | |

EP 0 513 162 B1

TABLE III

| Degrees 2-Theta | Interplanar d-Spacing (A) | $I/I_o$ |
|---|---|---|
| 2.80 | 31.55 | 25 |
| 4.02 | 21.98 | 10 |
| 7.10 | 12.45 | 96 |
| 7.95 | 11.12 | 47 |
| 10.00 | 8.85 | 51 |
| 12.90 | 6.86 | 11 |
| 14.34 | 6.18 | 42 |
| 14.72 | 6.02 | 15 |
| 15.90 | 5.57 | 20 |
| 17.81 | 4.98 | 5 |
| 20.20 | 4.40 | 20 |
| 20.91 | 4.25 | 5 |
| 21.59 | 4.12 | 20 |
| 21.92 | 4.06 | 13 |
| 22.67 | 3.92 | 30 |
| 23.70 | 3.75 | 13 |
| 24.97 | 3.57 | 15 |
| 25.01 | 3.56 | 20 |
| 26.00 | 3.43 | 100 |
| 26.69 | 3.31 | 14 |
| 27.75 | 3.21 | 15 |
| 28.52 | 3.13 | 10 |
| 29.01 | 3.08 | 5 |
| 29.71 | 3.01 | 5 |
| 31.61 | 2.830 | 5 |
| 32.21 | 2.779 | 5 |
| 33.35 | 2.687 | 5 |
| 34.61 | 2.592 | 5 |

EXAMPLE 2

A portion of the calcined crystalline product of Example 1 was tested in the Alpha Test and was found to have an Alpha Value of 224.

EXAMPLES 3-5

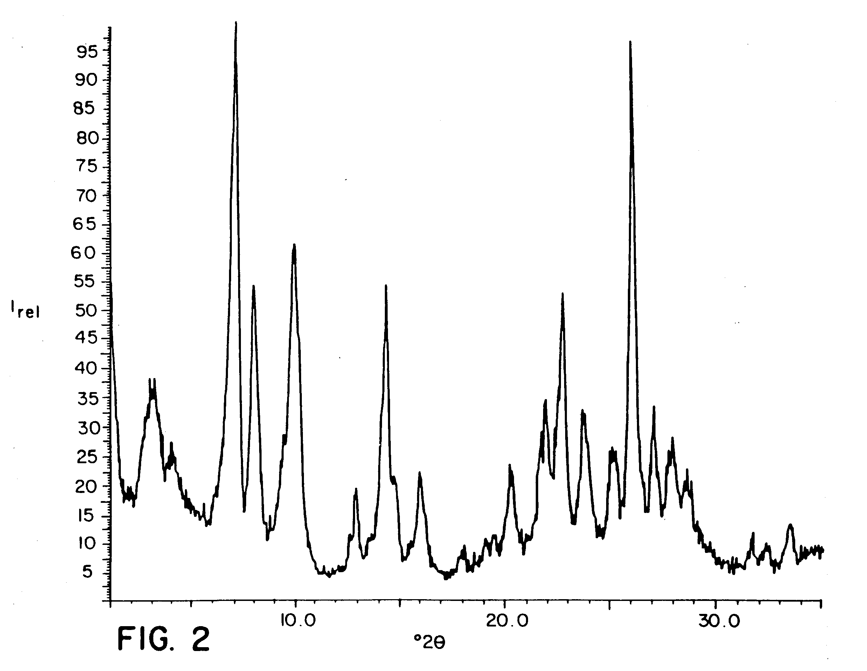

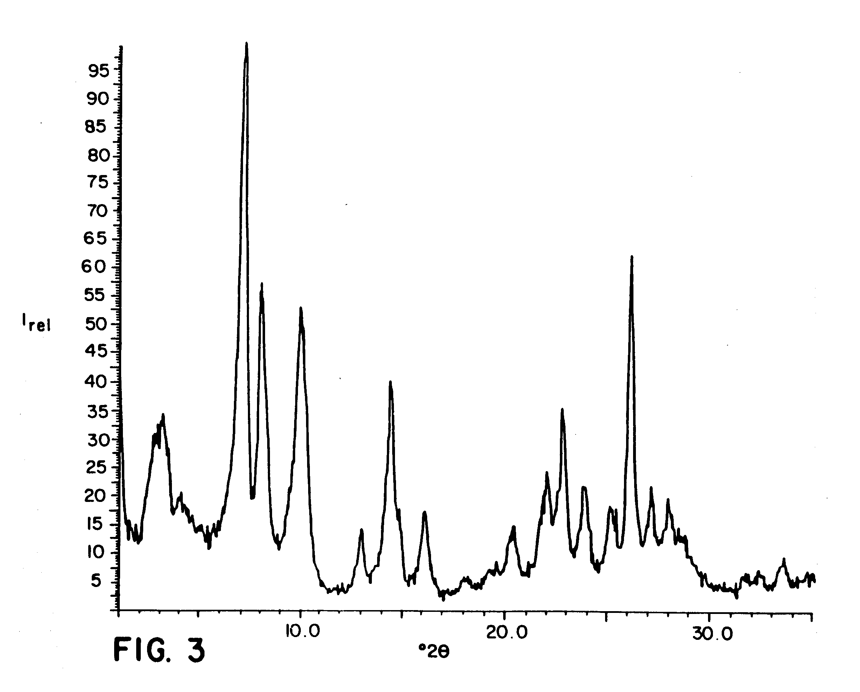

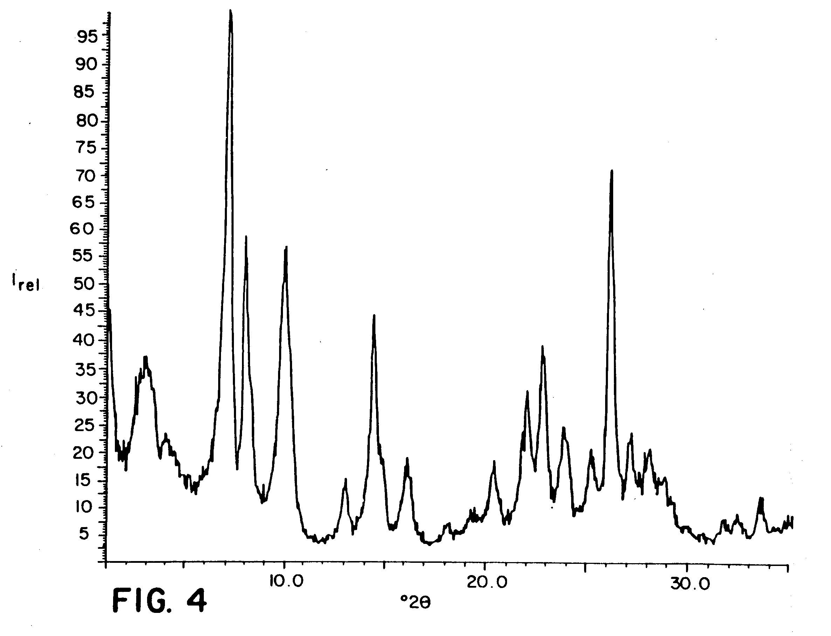

Three separate synthesis reaction mixtures were prepared with compositions indicated in Table VI. The mixtures were prepared with sodium aluminate, sodium hydroxide, Ultrasil, hexamethyleneimine (R) and water. The mixtures were maintained at 150°C, 143°C and 150°C, respectively, for 7, 8 ad 6 days respectively in stainless steel autoclaves at autogenous pressure. Solids were separated from any unreacted components by filtration and then water washed, followed by drying at 120°C. The product crystals were analyzed by X-ray diffraction, sorption, surface area and chemical analyses and the results are presented in Table IV and Figures 2, 3 and 4. The sorption and surface area measurements were of the calcined product.

TABLE IV

| Example | 3 | 4 | 5 |
|---|---|---|---|
| Synthesis Mixture, mole ratios | | | |
| $SiO_2/Al_2O_3$ | 30.0 | 30.0 | 30.0 |
| $OH^-/SiO_2$ | 0.18 | 0.18 | 0.18 |
| $H_2O/SiO_2$ | 19.4 | 19.4 | 44.9 |
| $Na/SiO_2$ | 0.18 | 0.18 | 0.18 |
| $R/SiO_2$ | 0.35 | 0.35 | 0.35 |
| Product Composition, Wt.% | | | |
| $SiO_2$ | 64.3 | 68.5 | 74.5 |
| $Al_2O_3$ | 4.85 | 5.58 | 4.87 |
| Na | 0.08 | 0.05 | 0.01 |
| N | 2.40 | 2.33 | 2.12 |
| Ash | 77.1 | 77.3 | 78.2 |
| $SiO_2/Al_2O_3$, mole ratio | 22.5 | 20.9 | 26.0 |
| Adsorption, Wt.% | | | |
| $H_2O$ | 14.9 | 13.6 | 14.6 |
| Cyclohexane | 12.5 | 12.2 | 13.6 |
| n-Hexane | 14.6 | 16.2 | 19.0 |
| Surface Area, $m^2/g$ | 481 | 492 | 487 |

EXAMPLE 6

Quantities of the calcined (538°C for 3 hours) crystalline silicate products of Examples 3, 4 and 5 were tested in the Alpha Test and found to have Alpha Values of 227, 180 and 187, respectively.

EXAMPLE 7

To demonstrate a further preparation of the present zeolite, 4.49 parts of hexamethyleneimine was added to a solution containing 1 part of sodium aluminate, 1 part of 50% NaOH solution and 44.19 parts of $H_2O$. To the combined solution were added 8.54 parts of Ultrasil silica. The mixture was crystallized with agitation at 145°C for 59 hours and the resultant product was water washed and dried at 120°C.

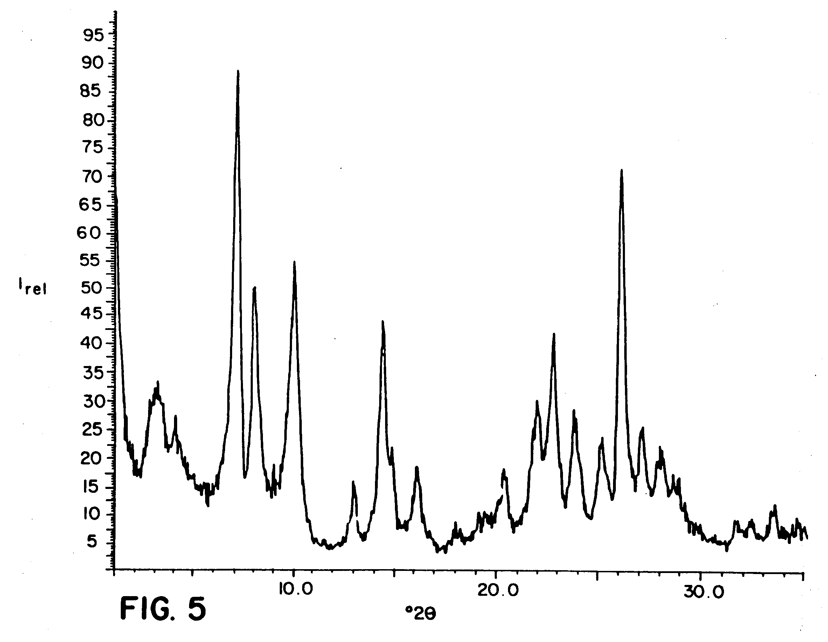

The X-ray diffraction pattern of the dried product crystals is presented in Figure 5. Product chemical composition, surface area and adsorption analyses results are set forth in Table V:

TABLE V

| Product Composition (uncalcined) | |
|---|---|
| C | 12.1 wt.% |
| N | 1.98 wt.% |
| Na | 640 ppm |
| $Al_2O_3$ | 5.0 wt.% |
| $SiO_2$ | 74.9 wt.% |
| $SiO_2/Al_2O_3$, mole ratio Cyclohexane | 25.4 9.1 |
| Adsorption, wt.% | |
| Cyclohexane | 9.1 |
| N-Hexane | 14.9 |
| $H_2O$ | 16.8 |
| Surface Area, $m^2/g$ | 479 |

12

EXAMPLE 8

25g grams of solid crystal product from Example 7 were calcined in a flowing nitrogen atmospheres at 538°C for 5 hours, followed by purging with 5% oxygen gas (balance $N_2$) for another 16 hours at 538°C.

Individual 3g samples of the calcined material were ion-exchanged with 100 ml of 0.1N TEABr, TPABr and $LaCl_3$ solution separately. Each exchange was carried out at ambient temperature for 24 hours and repeated three times. The exchanged samples were collected by filtration, water-washed to be halide-free and dried. The compositions of the exchanged samples are tabulated below demonstrating the exchange capacity of the present crystalline silicate for different ions.

| Exchange Ions | TEA | TPA | La |
|---|---|---|---|
| Ionic Composition, wt.% | | | |
| Na | 0.095 | 0.089 | 0.063 |
| N | 0.30 | 0.38 | 0.03 |
| C | 2.89 | 3.63 | - |
| La | - | - | 1.04 |

EXAMPLE 9

The La-exchanged sample from Example 8 was sized to 14 to 25 mesh and then calcined in air at 538°C for 3 hours. The calcined material had an Alpha Value of 173.

EXAMPLE 10

The calcined sample La-exchanged material from Example 9 was severely steamed at 649°C in 100% steam for 2 hours. The steamed sample had an Alpha Value of 22, demonstrating that the zeolite had very good stability under severe hydrothermal treatment.

EXAMPLE 11

This example illustrates the preparation of the present zeolite where X in the general formula, supra, is boron. Boric acid, 2.59 parts, was added to a solution containing 1 part of 45% KOH solution and 42.96 parts $H_2O$. To this was added 8.56 parts of Ultrasil silica, and the mixture was thoroughly homogenized. A 3.88 parts quantity of hexamethyleneimine was added to the mixture.

The reaction mixture had the following composition in mole ratios:

| | |
|---|---|
| $SiO_2/B_2O_3$ | = 6.1 |
| $OH^-/SiO_2$ | = 0.06 |
| $H_2O/SiO_2$ | = 19.0 |
| $K/SiO_2$ | = 0.06 |
| $R/SiO_2$ | = 0.30 |

where R is hexamethyleneimine.

The mixture was crystallized in a stainless steel reactor, with agitation, at 150°C for 8 days. The crystalline product was filtered, washed with water and dried at 120°C. A portion of the product was calcined for 6 hours at 540°C and found to have the following sorption capacities:

| | |
|---|---|
| $H_2O$ (12 Torr) | 11.7 wt.% |
| Cyclohexane (40 Torr) | 7.5 wt.% |
| n-Hexane (40 Torr) | 11.4 wt.% |

The surface area of the calcined crystalline material was measured (BET) to be 405m²/g.

The chemical composition of the uncalcined material was determined to be as follows:

| N | 1.94 wt.% |
| Na | 175 ppm |
| K | 0.60 wt.% |
| Boron | 1.04 wt.% |
| $Al_2O_3$ | 920 ppm |
| $SiO_2$ | 75.9 wt.% |
| Ash | 74.11 wt.% |
| $SiO_2/Al_2O_3$, molar ratio = 1406 | |
| $SiO_2/(Al + B)_2O_3$, molar ratio = 25.8 | |

EXAMPLE 12

A portion of the calcined crystalline product of Example 11 was treated with $NH_4Cl$ and again calcined. The final crystalline product was tested in the Alpha Test and found to have an Alpha Value of 1.

EXAMPLE 13

This example illustrates another preparation of the zeolite in which X of the general formula, supra, is boron. Boric acid, 2.23 parts, was added to a solution of 1 part of 50% NaOH solution and 73.89 parts $H_2O$. To this solution was added 15.29 parts of HiSil silica followed by 6.69 parts of hexamethyleneimine. The reaction mixture had the following composition in mole ratios:

| $SiO_2/B_2O_3$ | = 12.3 |
| $OH^-/SiO_2$ | = 0.056 |
| $H_2O/SiO_2$ | = 18.6 |
| $K/SiO_2$ | = 0.056 |
| $R/SiO_2$ | = 0.30 |

where R is hexamethyleneimine.

The mixture was crystallized in a stainless steel reactor, with agitation, at 300°C for 9 days. The crystalline product was filtered, washed with water and dried at 120°C. The sorption capacities of the calcined material (6 hours at 540°C) were measured:

| $H_2O$ | 14.4 wt.% |
| Cyclohexane | 4.6 wt.% |
| n-Hexane | 14.0 wt.% |

The surface area of the calcined crystalline material was measured to be 438m²/g.

The chemical composition of the uncalcined material was determined to be as follows:

| Component | Wt.% |
| --- | --- |
| N | 2.48 |
| Na | 0.06 |
| Boron | 0.83 |
| $Al_2O_3$ | 0.50 |
| $SiO_2$ | 73.4 |
| $SiO_2/Al_2O_3$, molar ratio = 249 | |
| $SiO_2/(Al + B)_2O_3$, molar ratio = 28.2 | |

14

EXAMPLE 14

A portion of the calcined crystalline product of Example 13 was tested in the Alpha Test and found to have an Alpha Value of 5.

EXAMPLES 15-19 AND COMPARATIVE EXAMPLES 20-26

Examples 15 to 19 illustrate the conversion of Udex raffinate whose composition is set forth above over the zeolite of the invention to provide a product mixture containing substantial amounts of BTX aromatics together with other aromatic and aliphatic hydrocarbons. Comparative Examples 20 to 26 illustrate the use of unbound ZSM-5 for conversion of the same feedstock under the same conditions.

The zeolite used in Examples 15-19 was produced by adding a 4.49 parts quantity of hexamethyleneimine to a mixture containing 1.00 parts sodium aluminate, 1.00 parts 50% NaOH, 8.54 parts Ultrasil VN3 and 44.19 parts deionized $H_2O$. The reaction mixture was heated to 143°C (290°F) and stirred in an autoclave at that temperature for crystallization. After full crystallinity was achieved, the majority of the hexamethyleneimine was removed from the autoclave by controlled distillation and the zeolite crystals separated from the remaining liquid by filtration, washed with deionized $H_2O$ and dried. The zeolite was then activated by calcining in nitrogen at 540°C (1000°F), followed by aqueous ammonium nitrate exchange in air at 540°C (1000°F).present in the hydrogen form after $N_2$/air calcination, ammonium exchange, and a second calcination in air. The zeolite was then tabletted, crushed and sized to 30/40 mesh and loaded into the reactor, a fixed-bed downflow reaction at a constant zeolite loading of 0.5 gm. The resultant catalyst had the following properties (Table VI):

TABLE VI

| Properties of the Conversion Catalyst of the Invention | |
|---|---|
| Surface Area (BET), $m^2$/g | 503 |
| $SiO_2$/$Al_2O_3$ (molar) | 27 |
| Na, ppm | 495 |
| Alpha $H_2O$ | 693 15.0 |
| Sorption Properties, wt.% | |
| $H_2O$ | 15.0 |
| $CyC_6$ | 12.5 |
| $n$-$C_6$ | 16.0 |
| Ash at 1000°C, wt.% | 99.05 |

Zeolite ZSM-5 which was used in Examples 20 to 26 ($SiO_2$/$Al_2O_3$ = 55) was prepared in a conventional manner.

Reaction conditions for Examples 15 to 26 were 538°C, 100 kPa (1 atm) and 2-30 WHSV.

The analyses of the conversion products of Examples 15-26 is set forth in Table VII as follows:

TABLE VII

| Example | 15 | 16 | 17 | 18 | 19 | 20 | 21 | 22 | 23 | 24 | 25 | 26 |
|---|---|---|---|---|---|---|---|---|---|---|---|---|
| TOS, hr | 1 | 3 | 4 | 7 | 9 | 1 | 2 | 4 | 5 | 6 | 8 | 10 |
| **Product Analysis, wt.%** | | | | | | | | | | | | |
| Hydrogen | 0.49 | 0.62 | 0.87 | 1.33 | 1.58 | 0.55 | 0.59 | 0.38 | 0.83 | 0.11 | 0.52 | 0.11 |
| Methane | 1.40 | 2.18 | 2.27 | 2.82 | 3.48 | 1.03 | 0.95 | 1.80 | 1.45 | 3.07 | 2.76 | 5.09 |
| Ethane | 2.58 | 3.82 | 4.00 | 4.59 | 5.60 | 2.20 | 2.17 | 3.58 | 3.06 | 5.87 | 5.22 | 8.41 |
| Ethylene | 3.11 | 3.59 | 3.36 | 2.71 | 2.28 | 3.23 | 3.21 | 4.26 | 3.57 | 4.93 | 4.56 | 4.77 |
| Propane | 9.97 | 14.24 | 15.05 | 15.60 | 16.36 | 7.51 | 7.19 | 12.02 | 10.22 | 18.34 | 16.96 | 25.10 |
| Propylene | 7.95 | 7.54 | 6.43 | 4.48 | 3.39 | 7.76 | 7.77 | 9.33 | 7.77 | 9.59 | 8.92 | 7.55 |
| Butanes | 8.72 | 9.57 | 7.75 | 6.60 | 5.99 | 6.27 | 6.17 | 9.44 | 7.94 | 12.42 | 11.63 | 13.17 |
| Butenes | 4.60 | 4.53 | 3.80 | 2.44 | 2.09 | 4.90 | 4.92 | 5.50 | 4.89 | 4.99 | 5.24 | 4.15 |
| Pentanes | 4.97 | 4.15 | 3.46 | 2.40 | 2.01 | 4.92 | 4.87 | 4.98 | 4.60 | 4.37 | 4.49 | 3.30 |
| Pentenes | 1.48 | 1.42 | 1.15 | 0.80 | 0.61 | 1.81 | 1.84 | 1.79 | 1.73 | 1.54 | 1.60 | 1.12 |
| Hexanes | 27.43 | 20.30 | 20.44 | 16.16 | 14.37 | 33.74 | 33.63 | 25.27 | 26.17 | 16.08 | 16.12 | 7.95 |
| Hexenes | 2.85 | 2.24 | 1.98 | 2.20 | 1.67 | 0.60 | 0.76 | 0.40 | 0.52 | -0- | -0- | 0.18 |
| Heptanes | 13.27 | 10.22 | 10.05 | 10.79 | 9.02 | 14.72 | 14.90 | 10.32 | 11.26 | 6.57 | 5.73 | 3.20 |
| Heptenes | 0.73 | 0.67 | 0.69 | 1.35 | 1.01 | 0.89 | 0.92 | 0.76 | 0.81 | -0- | -0- | 0.37 |
| Benzene | 1.26 | 2.08 | 2.60 | 4.42 | 5.88 | 1.01 | 1.01 | 1.56 | 1.56 | 2.16 | 2.05 | 3.44 |
| Toluene | 4.43 | 5.90 | 7.07 | 10.28 | 13.28 | 4.61 | 4.61 | 6.25 | 6.46 | 6.13 | 6.95 | 7.78 |
| Ethylbenzene | 0.39 | 0.63 | 0.91 | 1.10 | 1.18 | 0.32 | 0.32 | 0.49 | 0.52 | 0.30 | 0.49 | 0.39 |
| Xylenes | 2.18 | 3.44 | 4.33 | 4.97 | 5.89 | 1.78 | 1.85 | 0.32 | 3.59 | 2.02 | 3.75 | 3.08 |
| Trimethylbenzenes | 1.16 | 1.63 | 3.79 | 2.47 | 2.08 | 1.12 | 1.11 | 1.16 | 2.00 | 0.90 | 1.84 | 0.74 |
| $C_{10}^{+}$ Aromatics | 1.03 | 1.24 | -0- | 2.66 | 2.22 | 1.03 | 1.20 | 0.39 | 1.05 | 0.62 | 1.18 | 0.34 |

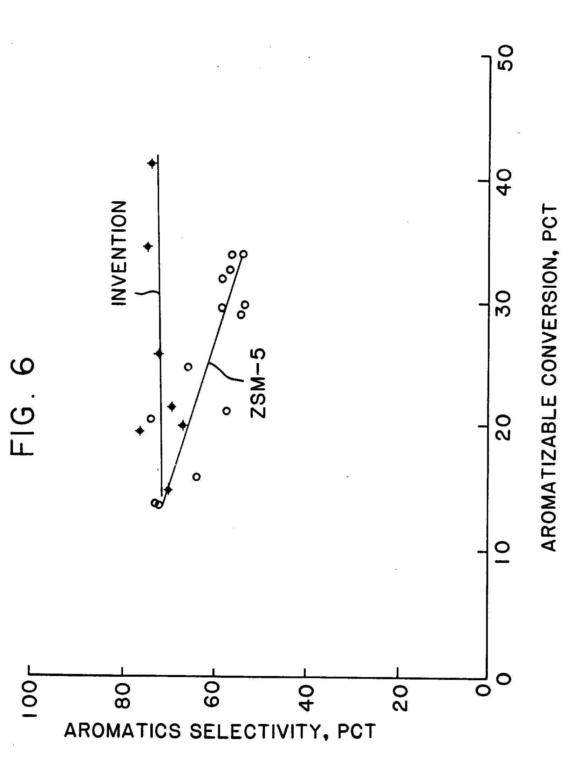

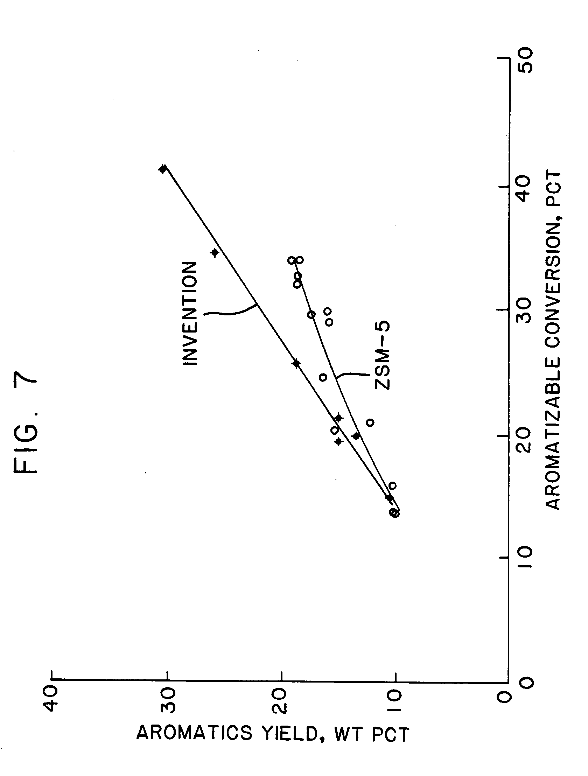

Figure 6 and 7 compare the aromatics selectivity and yield respectively for both ZSM-5 and the zeolite of the invention. At 25 to 40% aromatizable conversion, the zeolite of the invention shows significantly higher aromatics selectivity and yield than ZSM-5.

16

EXAMPLES 27-29

These examples illustrate the conversion of n-hexane over a catalyst containing the zeolite of the invention. The zeolite was produced by adding 4.49 parts, by weight, of hexamethyleneimine to a mixture containing 1.00 part sodium aluminate, 1.00 part 50% NaOH, 8.54 parts Ultrasil VN3 silica and 44.19 parts deionized $H_2O$. The reaction mixture was heat to 143°C (290°F) and stirred in an autoclave at that temperature for crystallization. After full crystallinity was achieved, the majority of the hexamethyleneimene was removed from the autoclave by controlled distillation and the zeolite crystals separated from the remaining liquid by filtration, washed with deionized $H_2O$ and dried.

A portion of the zeolite crystals was combined with $Al_2O_3$ to form a mixture of 65 parts, by weight, zeolite and 35 parts $Al_2O_3$. Water was added to this mixture to allow the resulting catalyst to be formed into extrudates. The catalyst was activated by calcining at 482°C (900°F) in 3v/v/min nitrogen for three hours, then treated with 50% vol.% air/50 vol.% $N_2$ at 3v/v/min, also at 482°C (900°F). The calcination was completed by raising the temperature to 540°C (1000°F) at 5°F/min and finally switching to 100% air (3v/v/min) and holding at 540]EC (1000°F) for three hours.

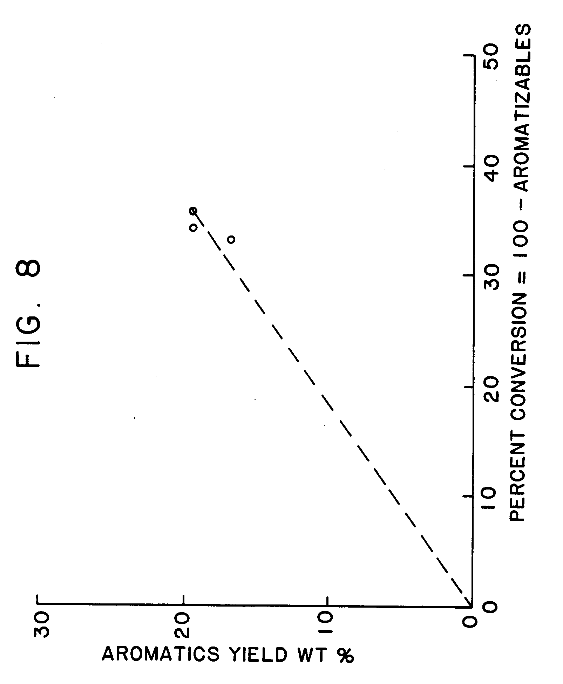

In each of the examples, the n-hexane conversion was effected at 540°C, 100 kPa (atmospheric) pressure and an LHSV of 0.6. Table VIII sets forth the results of the conversions and Figure 8 shows the yield of aromatics against the percentage of conversion for the combined results.

TABLE VIII

| Example | 27 | 28 | 29 |
|---|---|---|---|
| Time on Stream (hrs) | 6 | 30 | 54 |
| Products: | | | |
| $H_2$ | 0.67 | 0.58 | 0.55 |
| Methane | 3.97 | 3.24 | 3.71 |
| Ethane | 12.34 | 11.38 | 12.68 |
| Propane | 24.84 | 23.97 | 27.08 |
| Butanes | 11.05 | 9.27 | 8.90 |
| Pentanes | 1.52 | 1.04 | 0.93 |
| n-Hexane | 7.24 | 5.44 | 6.81 |
| $C_6$ + P + O | 1.29 | 2.51 | 2.75 |
| Ethylene | 4.42 | 4.63 | 4.72 |
| Propylene | 7.48 | 8.90 | 7.16 |
| $C_4$ Olefins | 4.51 | 7.07 | 5.61 |
| $C_5$ Olefins | 1.32 | 2.44 | 2.35 |
| Benzene | 2.30 | 2.55 | 2.52 |
| Toluene | 5.31 | 4.88 | 4.25 |
| $A_8$ | 4.65 | 5.15 | 4.86 |
| $A_9$ + | 7.09 | 6.95 | 5.12 |
| Aromatics selectivity | 54.25 | 57.19 | 50.54 |

EXAMPLE 30

This example illustrates the use of the present zeolite for n-hexane conversion after the zeolite has been steamed (100% steam) at 540°C (1000°F) for 48 hours. The reaction conditions were essentially the same as those employed in Examples 27-29.

The results of the conversion herein are set forth in Table IX as follows:

TABLE IX

| Time On Stream (hrs.) | 6 |
|---|---|
| | Products: |
| $H_2$ | 0.11 |
| Methane | 2.02 |
| Ethane | 4.96 |
| Propane | 8.13 |
| Butanes | 2.33 |
| Pentanes | 0.16 |
| n-Hexane | 52.30 |
| $C_6 + P + O$ | 2.36 |
| Ethylene | 4.02 |
| Propylene | 11.18 |
| $C_4$ Olefins | 1.96 |
| $C_5$ Olefins | 0.22 |
| Benzene | 2.44 |
| Toluene | 5.09 |
| $A_8$ | 1.94 |
| $A_9 +$ | 0.78 |
| Aromatics selectivity | 59.49 |

EXAMPLE 31

This example illustrates the preparation of a low acidity catlayst and its use in the conversion of a paraffin, n-hexane, to a mixture of olefin and aromatics.

10 gm of the hydrogen from the zeolite produced in Examples 15-19 were slurried in 100 ml water and to the resultant slurry were added 75 ml of 0.5 M $NaHCO_3$ followed by 50 ml water containing 200 mg Pt-$(NH_3)_4Cl_2$. This mixture was stirred overnight at ambient temperature at a pH of 8.8. The sodium and platinum-exchanged zeolite was filtered, washed, dried and calcined in oxygen by heating to 360°C at a rate of 0.5°C per minute and maintained at the final temperature for 1 hour. The low acidity calcined zeolite contained 1.5% Pt. 1.6% Na and 2.4% Al. The pH of the catalyst measured upon a slurry of 0.1 gm of the catalyst in 10 ml of distilled, deionized water was 7.0 - 7.1.

In a first run employing the catalyst of this example, n-hexane was converted at 538°C, 100 kPa (atmospheric pressure) and a 6:1 mole ratio of hydrogen to n-hexane to provide benzene with a selectivity of 11% at 55-60% conversion:

In a second run employing the catalyst, n-heptane was converted at 482°C, 790 kPa (100 psig) and a 5:1:1 mole ratio of $N_2:H_2:C_7$. Yields were 29% $C_3$ and 36% $C_4$ aliphatics and 4% benzene/toluene/xylene aromatics at 98.8% n-heptane conversion.

EXAMPLE 32

The low acidity calcined catalyst of Example 31 was further treated with 1M $NaHCO_3$ at room temperature and filtered without additional washing to render the catalyst essentially non-acidic. Thus, the pH of the catalyst measured upon a 0.1 gm slurry of the catalyst in distilled, deionized water was 11.3. The catalyst was employed in the conversion of n-hexane utilizing substantially the same procedures and reaction conditions as in Example 31. Analysis of the reaction product indicated 55% selectivity to benzene at 56% n-hexane conversion, a dramatic increase in selectivity for benzene compared with that obtained in Example 31.

EXAMPLE 33

This example illustrates the preparation of a low acidity catalyst for use in the paraffin conversion illustrated in Example 34, infra.

18

EP 0 513 162 B1

The zeolite produced in Examples 15-19 was subjected to repeated aqueous sodium nitrate exchange/540°C (1000°F) air calcination to prepare a low acidity catalyst having an Alpha Value of 2. This sodium form of the zeolite was then exchanged over 4 hours at ambient temperature with a 2.6 x $10^{-3}$M solution of $Pt(NH_3)_4Cl_2$ at a pH of 9 followed by washing with deionized water until the zeolite was free of chloride ion and drying thereafter. The zeolite contained 1.0 wt.% Pt. The platinum-loaded zeolite was then calcined in air, the temperature being increased at the rate of 1°C (2°F) per minute to a final temperature of 350°C (660°F), the zeolite being held at this temperature for 3 hours. Following a twice repeated exchange of the Pt/Na/zeolite with aqueous sodium nitrate and washing with deionized water, the catalyst was dried at 121°C (250°F).

EXAMPLE 34

This example compares the catalytic performance of the low acidity Pt/Na/zeolite catalyst composition of Example 33 with that of a commercial reforming catalyst, namely, $Pt/Re/Al_2O_3$ (containing 0.22 wt.% Pt and 0.44 wt.% Re).

In each run, 10cc (approximately 4 grams) of each of the aforesaid catalysts were employed. The catalyst bed was heated at a rate of 104°C/hr (220°F/hr) in 100 cc/min of flowing hydrogen to 510°C (950°F) and then held at this temperature for one hour. Conversion data for each catalyst were obtained at process conditions of 510°C (950°F), 790 kPa (100 psig), 3:1 $H_2$/HC mole rate and 3 WHSV with a 100% n-hexane feed.

The results of each run are set forth in Tables X and XI below and are graphically compared in Fig. 6.

TABLE X

| Dehydrocyclization of n-Hexane Using Catalyst of Invention | | | | | |
|---|---|---|---|---|---|
| Hours on Stream | 1 | 5 | 10 | 20 | 24 |
| $C_5$-, wt.% | | 71.6 | 68.5 | 64.8 | 62.6 |
| Benzene | 33.4 | 20.5 | 18.5 | 18.3 | 17.7 |
| $C_7$ + Aromatics | 2.3 | 1.7 | 1.5 | 1.3 | 1.3 |
| Total Aromatics | 35.7 | 22.2 | 20.0 | 19.6 | 19.0 |

TABLE XI

| Dehydrocyclization of n-Hexane Employing $Pt/Re/Al_2O_3$ Catalyst | | | |
|---|---|---|---|
| Hours on Stream | 3 | 28 | 44 |
| $C_5$-, wt.% | 49.6 | 41.5 | 36.0 |
| Benzene | 9.2 | 5.8 | 4.3 |
| $C_7$ + Aromatics | 4.3 | 4.2 | 3.9 |
| Total Aromatics | 13.5 | 10.0 | 8.2 |

As these data show, aromatics production with the low acidity Pt/Na/zeolite catalyst of the invention was initially greater than 33 wt.%, reducing to 20 wt.% after several hours on stream, compared with about a 10 wt.% production of aromatics in the case of the $Pt/Re/Al_2O_3$ catalyst.

**Claims**

1. A process for dehydrogenation and/or dehydrocyclization of a feedstock containing at least one $C_2$-$C_{12}$ aliphatic hydrocarbon comprising contacting the feedstock with a conversion catalyst comprising a synthetic porous crystalline zeolite having, in its calcined form, an X-ray diffraction pattern including the values listed in Table I of the specification.

2. The process of Claim 1 wherein the synthetic porous crystalline zeolite is, in its calcined form, an X-ray diffraction pattern including the values listed in Table II of the specification.

3. The process of Claim 1 wherein the zeolite has a composition comprising the molar relationship

$X_2O_3:(n)YO_2,$

wherein n is at least about 10, X is a trivalent element and Y is a tetravalent element.

4. The process of Claim 5 wherein X comprises aluminum and Y comprises silicon.

5. The process of Claim 1 wherein the zeolite has equilibrium adsorption capacities of greater than 4.5 wt.% for cyclohexane vapor and greater than 10 wt.% for n-hexane vapor.

6. The process of Claim 1 wherein the zeolite has an Alpha Value not greater than 150 and the catalyst further comprises a Group VIII metal species.

7. The process of Claim 1 wherein the zeolite has essentially no acidity and the catalyst further comprises a Group VIII metal species.

8. The process of claim 6 or claim 7 wherein the zeolite is associated therewith at least one element selected from tin, indium, thallium, lead and sulfur.

9. The process of Claim 1 wherein the feedstock contains at least 20% by weight of at least one $C_2$-$C_{12}$ aliphatic hydrocarbon.

10. The process of Claim 1 wherein the feedstock contains at least 50% by weight of at least one $C_2$-$C_{12}$ aliphatic hydrocarbon.

11. The process of Claim 1 carried out at a temperature of 316 to 760°C (600°F to 1400°F), a pressure of from 100 to 2860 kPa (atmospheric to 400 psig) and an LHSV of 0.1 to 100.

12. The process of Claim 1 carried out at a temperature of 400 - 680°C (750 to 1250°F), a pressure of 445 to 1825 kPa (50 to 250 psig) and an LHSV of 0.5 to 5.

**Patentansprüche**

1. Verfahren zur Dehydrierung und/oder Dehydrocyclisierung eines Ausgangsmaterials, das mindestens einen aliphatischen $C_2$-$C_{12}$-Kohlenwasserstoff enthält, das den Kontakt des Ausgangsmaterials mit einem Umwandlungskatalysator umfaßt, der einen synthetischen porösen kristallinen Zeolith umfaßt, der in seiner kalzinierten Form ein Röntgenbeugungsdiagramm mit den in Tabelle I der Beschreibung aufgeführten Werten aufweist.

2. Verfahren nach Anspruch 1, wobei der synthetische poröse kristalline Zeolith in seiner kalzinierten Form ein Röntgenbeugungsdiagramm hat, das die in Tabelle II der Beschreibung aufgeführten Werte enthält.

3. Verfahren nach Anspruch 1, wobei der Zeolith eine Zusammensetzung aufweist, die das Molverhältnis hat:

$X_2O_3:(n)YO_2,$

worin n mindestens etwa 10 beträgt, X ein dreiwertiges Element und Y ein vierwertiges Element sind.

4. Verfahren nach Anspruch 5, wobei X Aluminium und Y Silicium umfassen.

5. Verfahren nach Anspruch 1, wobei der Zeolith Gleichgewichts-Adsorptionskapazitätenvon mehr als 4,5 Gew.-% für Cyclohexandampf und mehr als 10 Gew.-% für n-Hexandampf aufweist.

6. Verfahren nach Anspruch 1, wobei der Zeolith einen $\alpha$-Wert von nicht mehr als 150 aufweist und der Katalysator außerdem eine Metallart der Gruppe VIII umfaßt.

**7.** Verfahren nach Anspruch 1, wobei der Zeolith im wesentlichen keine Acidität aufweist und der Katalysator außerdem Metallarten der Gruppe VIII umfaßt.

**8.** Verfahren nach Anspruch 6 oder 7, wobei mit dem Zeolith zumindest ein Element verbunden ist, das aus Zinn, Indium, Thallium, Blei und Schwefel ausgewählt ist.

**9.** Verfahren nach Anspruch 1, wobei das Ausgangsmaterial mindestens 20 Gew.-% von mindestens einem aliphatischen $C_2$-$C_{12}$-Kohlenwasserstoff enthält.

**10.** Verfahren nach Anspruch 1, wobei das Ausgangsmaterial mindestestens 50 Gew.-% mindestens von mindestens einem aliphatischen $C_2$-$C_{12}$-Kohlenwasserstoff enthält.

**11.** Verfahren nach Anspruch 1, das bei einer Temperatur von 316 bis 760°C (600 bis 1400°F), einem Druck von 100 bis 2860 kPa (atmosphärischer Druck bis 400 psig) und einer LHSV von 0,1 bis 100 durchgeführt wird.

**12.** Verfahren nach Anspruch 1, das bei einer Temperatur von 400-680°C (750 bis 1250°F), einem Druck von 445 bis 1825 kPa (50 bis 250 psig) und einer LHSV von 0,5 bis 5 durchgeführt wird.

## Revendications

**1.** Procédé de déshydrogénation et/ou de déshydrocyclisation d'une charge contenant au moins un hydrocarbure aliphatique en $C_2$ à $C_{12}$, comprenant la mise au contact de la charge d'alimentation avec un catalyseur de conversion comprenant une zéolite cristalline poreuse synthétique sous sa forme calcinée, dont le spectre de diffraction aux rayons X a les valeurs caractéristiques énumérées dans le tableau I de la description.

**2.** Procédé selon la revendication 1, dans lequel la zéolite cristalline poreuse synthétique présente, sous sa forme calcinée, un spectre de diffraction aux rayons-X ayant les valeurs énumérées dans le tableau II de la description.

**3.** Procédé selon la revendication 1, dans lequel la zéolite a la composition exprimée en terme de molarité:

$$X_2O_3/(n)YO_2$$

dans laquelle:
n       est au moins égal à 10 environ;
X       est un élément trivalent; et
Y       est un élément tétravalent.

**4.** Procédé selon la revendication 3, dans lequel X est l'aluminium et Y est le silicium.

**5.** Procédé selon la revendication 1, dans lequel la zéolite présente une capacité d'adsorption à l'équilibre supérieure à 4,5% en poids pour le cyclohexane sous forme vapeur, et supérieure à environ 10% en poids pour le n-hexane vapeur.

**6.** Procédé selon la revendication 1, dans lequel la zéolite présente une valeur Alpha non supérieure à 150 et dans lequel le catalyseur renferme en outre des espèces métalliques appartenant au groupe VIII.

**7.** Procédé selon la revendication 1, dans lequel la zéolite n'a pratiquement pas d'acidité et le catalyseur renferme en outre des espèces métalliques du groupe VIII.

**8.** Procédé selon la revendication 6 ou la revendication 7, dans lequel la zéolite est associée avec au moins un élément choisi parmi étain, indium, thallium, plomb et soufre.

**9.** Procédé selon la revendication 1, dans lequel la charge d'alimentation renferme au moins 20% en poids d'au moins un hydrocarbure aliphatique en $C_2$ à $C_{12}$.

10. Procédé selon la revendication 1, dans lequel la charge d'alimentation renferme au moins 50% en poids d'au moins un hydrocarbure aliphatique en $C_2$ à $C_{12}$.

11. Procédé selon la revendication 1, mis en oeuvre à une température de 316 à 760°C (600°F à 1400°F), sous une pression de 100 à 2860 kPa (pression atmosphérique à 400 psig) et une LHSV de 0,1 à 100.

12. Procédé selon la revendication 1, mis en oeuvre à une température de 400 à 680°C (750°F à 1250°F), sous une pression de 445 à 1825 kPa (50 à 250 psig) et une LHSV de 0,5 à 5.

FIG. 1

$I_{rel}$

°2θ

EP 0 513 162 B1

FIG. 2

EP 0 513 162 B1

EP 0 513 162 B1

FIG. 3

FIG. 4

FIG. 5

FIG. 6

FIG. 7

## FIG. 8

AROMATICS YIELD WT %

PERCENT CONVERSION = 100 − AROMATIZABLES